# EUROPEAN PATENT APPLICATION

(11) **EP 1 317 915 A1**
(43) Date of publication of application: **11.06.2003**
(21) Application number: 01963594.5
(22) Date of filing: 11.09.2001
(51) Int. Cl.: A61K 7/06, A61K 45/06, A61K 35/78, A61K 38/06, A61K 31/366, A61P 1/16, A61P 13/12, G01N 33/15, G01N 33/50

(54) **HAIR NOURISHMENTS AND METHOD OF SCREENING THE SAME**

(30) Priority: 11.09.2000 JP 2000275639; 11.09.2000 JP 2000275703; 29.09.2000 JP 2000299341
(71) Applicant: SHISEIDO COMPANY, LTD., Chuo-ku, Tokyo 104-8010 (JP)
(72) Inventor: HIBINO, Toshihiko, C/O SHISEIDO RESEARCH CENTER, Yokohama-shi, Kanagawa 236-0004 (JP); TSUJI, Yumiko, C/O SHISEIDO RESEARCH CENTER, Yokohama-shi, Kanagawa 236-0004 (JP); SOMA, Tsutomu, C/O SHISEIDO RESEARCH CENTER, Yokohama-shi, Kanagawa 236-0004 (JP); DENDA, Sumiko, C/O SHISEIDO RESEARCH CENTER, Yokohama-shi, Kanagawa 236-0004 (JP); NAKANISHI, Jotaro, C/O SHISEIDO RESEARCH CENTER, Yokohama-shi, Kanagawa 236-0004 (JP); TAKAHASHI, Tadahito, C/O SHISEIDO RESEARCH CENTER, Yokohama-shi, Kanagawa 224-8558 (JP); UMISHIO, Kenichi, C/O SHISEIDO RESEARCH CENTER, Yokohama-shi, Kanagawa 224-8558 (JP); KOBAYASHI, Koji, C/O SHISEIDO RESEARCH CENTER, Yokohama-shi, Kanagawa 224-8558 (JP)
(74) Representative: Santarelli
(86) International application number: JP0107888
(87) International publication number: WO02022088

(57) **Abstract**

There is provided a hair tonic comprising at least two of an inhibitor against 5α reductase type 2, an inhibitor against transforming growth factor β2, and an inhibitor against caspase-3; a method comprising screening for at least two of inhibition against 5α reductase type 2, inhibition against transforming growth factor β2, and inhibition against caspase-3, or comprising screening for inhibition against caspase-3 and then for apoptosis inhibition, and a hair loss inhibitor having the effect of inhibiting caspase-3, and a hair loss inhibitor having the effect of inhibiting caspase-3 and of inhibiting apoptosis.

## Description

### Field of Invention

The present invention relates to hair tonics and methods of screening them and, more specifically, it relates to hair tonics comprising a combination of two or more hair growth phase extenders of the hair cycle, and methods of selecting them.

The present invention also relates to aspartase-3 inhibitors, and more specifically to caspatase inhibitors comprising, as active ingredients, plant extracts having an activity of extending the hair growth phase of the hair cycle, hair tonics comprising them and, in particular, to hair loss inhibitors comprising them.

The present invention also relates to preventive or therapeutic compositions for the hair, etc., comprising, as an active ingredient, isocoumarin and specifically phyllodulcin. More specifically, it relates to hair tonic cosmetics, inhibitors of transition into the regression phase, therapeutic agents for liver cirrhosis or therapeutic agents for nephritis comprising as an active ingredient an isocoumarin, specifically phyllodulcin. More preferably, it relates to hair tonic cosmetics or inhibitors of transition into the regression phase comprising as an active ingredient isocoumarin, dihydroisocoumaris among others and, specifically, phyllodulcin.

### Background Art

It is known that male hormones and apoptosis are involved in hair loss in men. It is generally known that when the apoptosis signal is sent to the cell, a cascade consisting of a plurality of caspases is activated leading to the formation, from the precursor caspase-3, of caspase-3, which cleaves cytoskeletal proteins and inhibitors of caspase activated DNAse (ICAD), eventually leading to irreversible cell death (apoptosis).

However, the process of hair loss from the involvement of male hormones leading to caspase-mediated apoptosis has not been elucidated, nor has the relationship of hair loss and caspase-3 been elucidated. Thus, there are no known hair tonics that were reasonably designed based on the elucidated mechanism of hair loss.

Cosmetics for hair, commonly termed as hair tonic cosmetics or hair growth agents, are expected to promote the extension of hair, for which purpose drugs have been contained that enter the hair root, dilate blood vessels, promote blood circulation, stimulate hair papillae, aid in the formation of hair, and promote hairing. Drugs contained in the hair tonic cosmetics include, by action, ingredients that have the effect of blood circulation promotion, local stimulation, hair follicle revitalization, anti-male hormone, anti-seborrhea, keratiolysis, sterilization, or are anti-inflammation.

Ingredients that have been blended with potential effects include specifically swertia extracts, vitamin E, capsicum tincture, vitamin B6, hinokitiol, estradiol, sulfur, salicylic acid, menthol, glycyrrhizin, panthothenic acid and the like.

Methods for evaluating hair tonic cosmetics include, as existing methods, those that utilize animals such as mice, rabbits and monkeys, those that utilize humans, those that utilize tissue cultures and the like. Among them, those that utilize humans are a direct method in which a test substance is applied on the human head and the extension of hair is observed, and in those that utilize tissue cultures hair follicles isolated from the skin are cultured in the presence of a test substance and then the degree of extension of the cultured hair is measured.

As to these conventional methods, there are problems in that the former methods of using humans require a long time to obtain results, and the latter methods are complicated in handling since they require the use of microscopes to obtain results, and cannot assay a large volume of samples at one time. Thus, there has been a need for a screening method that can be performed in a short period of time and in simple procedures, and that can assay a large volume of samples at one time.

From the foregoing, the present inventors have developed a novel screening method that can be applied to the screening of hair tonic cosmetics and that can be performed in a short period of time and in simple procedures, and that can assay a large volume of samples at one time, and have applied for patent (Patent Application No. 11-86406).

### Disclosure of the Invention

The present invention provides hair tonics that were reasonably designed based on the elucidation of hair loss mechanism, and methods of screening them.

After intensive and extensive research to solve the above problems, the present inventors have elucidated a series of cascade (the regression phase cascade) in which 5α reductase type 2 (5αR-II) has been expressed on the sinciput region of patients with maple pattern alopecia, which enzyme produces a potent male hormone, DHT, from testosterone at the sinciput, and this male hormone acts on the hair papilla cells to enhance the production of transforming growth factor β2 (TGF β2), which TGF β2 activates caspases in the follicular epithelial cells thereby to induce apoptosis, by which transition from the growth phase to the regression phase of the hair cycle, i.e., miniaturization or fluffing is promoted leading to hair loss.

Based on this elucidation, the present inventors have found a novel hair tonic and a method of screening it.

Thus, the present invention provides a hair tonic comprising a combination of at least two of the following:
(a) an inhibitor against 5α reductase type 2 (5αR-II);
(b) an inhibitor against transforming growth factor β2 (TGF β2); and
(c) an inhibitor against caspase-3.

It is preferred to combine (b) an inhibitor against transforming growth factor β2 (TGF β2) with (c) an inhibitor against caspase-3 that is present downstream in the cascade.

The present invention also provides a method of screening an inhibitor of male patterned hair loss comprising a combination of at least two selections of the following:
(a) selection of an inhibitor against 5α reductase type 2 (5αR-II);
(b) selection of an inhibitor against transforming growth factor β2 (TGF β2); and
(c) selection of an inhibitor against caspase-3.

It is preferred to combine (b) selection of an inhibitor against transforming growth factor β2 (TGF β2) with (c) selection of an inhibitor against caspase-3 that are present in the downstream of the cascade.

However, though the above method is preferred for the testing of a large volume of test substances as the primary screening of hair loss inhibitors, it lacks precision required for the final selection of the desired substances. Thus, it is preferred to select test substances by the primary screening in the above method followed by the selection of more direct apoptosis inhibitors.

Thus the present invention also provides a method of screening an inhibitor of male patterned hair loss comprising selecting inhibitors against caspase-3, and selecting, from the substances selected as above, substances that inhibit apoptosis in the cultured cells of mouse epithelial keratinocytes.

The present invention also provides a novel caspase inhibitor that is useful as an ingredient of hair tonics, especially of hair loss inhibitors.

After intensive and extensive research to solve the above problems, the present inventors have elucidated a series of cascade in which 5α reductase type 2 (5αR-II) enhances the production of a male hormone (DHT), and this male hormone in turn enhances the production of transforming growth factor β2 (TGF β2), which TGF β2 activates caspases thereby to promote transition from the growth phase to the regression phase of the hair cycle, i.e., miniaturization or fluffing is promoted leading to hair loss.

Thus, the screening of hair loss inhibitors can be performed by the selection of either of caspases, especially the selection of inhibitors against caspase-3 present in the furthermost downstream of the cascade.

Thus, the present invention provides a novel inhibitor against caspase-3, and a hair tonic, especially a hair loss inhibitor, comprising the same as an active ingredient. As such caspase-3 inhibitors, there can be illustrated extracts of Indigofera tinctoria Linn, extracts of Cassia fistula, extracts of Lamium album, extracts of tormentilla, extracts of grape leaves, extracts of linden, extracts of Filipendula multijuga, extracts of Cuachalalate, extracts of black tea, extracts of oolong tea, extracts of Coix lacryma-jobi, and extracts of Lithospermum erythorhizon siebolt Zuccarini.

However, in the above method, preferably the hair loss inhibitors have not only a caspase-3 inhibiting activity but also a more direct effect of inhibiting apoptosis.

Thus, the present invention also provides a hair tonic ingredient that has an activity of inhibiting caspase-3 and furthermore an activity of inhibiting apoptosis in cultured cells of mouse epithelial keratinocytes. As such ingredients, there can be illustrated extracts of Cuachalalate, extracts of Filipendula multijuga, extracts of black tea, extracts of oolong tea, extracts of Coix lacryma-jobi, and extracts of Lithospermum erythorhizon siebolt Zuccarini.

Another novel screening method of the present invention was developed based on the discovery by the present inventors that a hair growing effect can be obtained in that tumor growth factor (transforming growth factor β2; TGF β2) promotes transition from the growth phase to the regression phase of the hair growth cycle, and an antagonizing substance (antagonist) of TGF-β2 inhibits the transition to the regression phase.

The present inventors have further confirmed that when human papilla cells are cultured in the presence or absence of TGF-β2 and an antagonist against TGF-β2, the amount produced of plasminogen activator inhibitor-1 (PAI-1) becomes decreased in the presence of the TGF-β2 antagonist as compared to the amount of PAI-1 produced in the absence of the antagonist. The present invention was developed based on the discovery of this new finding.

In the course of confirming the effectiveness of this screening method, various crude drugs were screened and it was confirmed that many of them had an antagonistic effect against TGF-β2, and they were applied for patent as hair tonic cosmetics together with the above method of screening. Among them, it was also confirmed, extracts of sweet hydrangea leaves had the most potent antagonistic effect against TGF-β2.

The extracts of sweet hydrangea leaves that were found to have the most potent antagonistic effect (the effect of inhibiting TGF-β2 with PAI-1 as an index, the extending effect by the human follicle organ culture) were further investigated to thereby identify the active ingredient. Thus, the identity of effect was confirmed to be phyllodulcin, one of the isocoumarins, especially dihydroisocoumarins.

### Brief Explanation of the Drawings

Fig. 1 is an electrophoretogram showing that 5αR-II is only expressed on the sinciput region of a patient with male patterned hair loss.
Fig. 2 is a photograph showing that transition to the regression phase is promoted when human hair follicle is organ-cultured for 6 days in the presence of TGF-β2.
Fig. 3 is a photograph showing the distribution of TGF-β2 during the growth phase and the regression phase of human hair follicle. Strong immunostaining of TGF-β2 is observed at the arrow.
Fig. 4 is a photograph showing changes (immunostaining) in TGF-β2 from the growth phase to the regression phase. Strong immunostaining of TGF-β2 is observed at the boundary (arrow) of the dermal papilla and the hair follicle.
Fig. 5 is a photograph showing the distribution of TGF-β2 during the early regression phase of human hair follicle.
Fig. 6 is a graph showing the effect of anti-TGF-β2 neutralizing antibody on the extension of hair when human hair follicles are subjected to organ culture.
Fig. 7 is a graph showing the effect of fetuin on the extension of hair when human hair follicles are subjected to organ culture.
Fig. 8 is a photograph showing that hair matrix cells in the periphery of dermal papilla in the regression phase are developing apoptosis (activation of caspase-3).
Fig. 9 is a photograph showing the distribution of the precursor caspase-3 throughout the hair cycle of human hair.
Fig. 10 is a graph showing the effect of z-VAD-fmk on the extension of hair by the human hair follicle organ culture method.

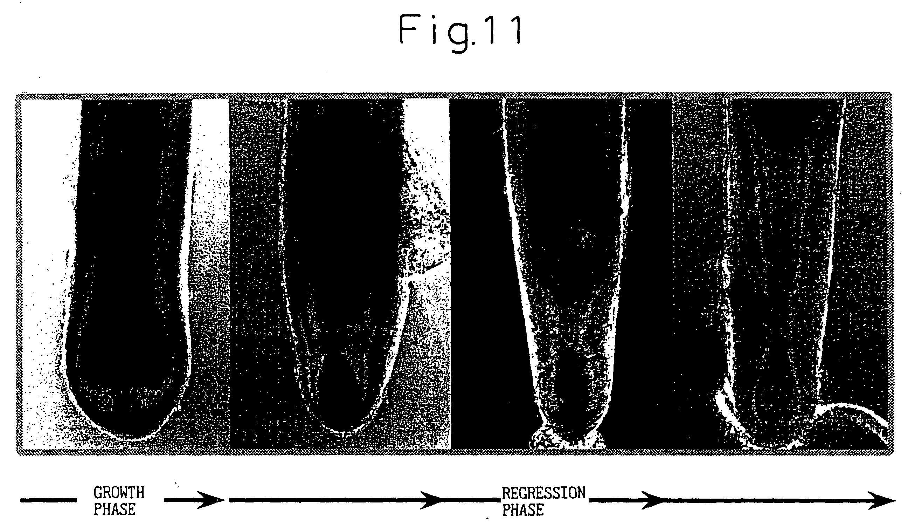

Fig. 11 is a photograph showing the morphological changes in the hair bulbs during natural transition from the growth phase to the regression phase.
Fig. 12 is a graph showing the effect of inhibiting caspase-3 by the extracts of various plants.
Fig. 13 is a photograph showing that the morphology of hair follicle is well retained in the coexistence of fetuin, a TGF-β2 inhibitor, and z-VAD-fmk, a caspase-3 inhibitor.
Fig. 14 shows that apoptosis is inhibited by the addition of Cuachalalate extracts in a system in which Pam212 cells develop apoptosis in the presence of TNF-α and CHX, and is a photograph showing the morphology of an organism.
Fig. 15 is a photograph showing that the activation of the precursor caspase-3 is inhibited by the addition of Cuachalalate extracts or VAD-fmk in Pam212 cells cultured in the presence of TNF-α and CHX.
Fig. 16 shows chemical formulas of existing ingredients in extracts of sweet hydrangea leaves.
Fig. 17 shows the result of investigation of phyllodulcin with regard to the effect by TGF-β2 of inhibiting the enhanced expression of PAI-1.
Fig. 18 shows the result of determination of the Alamar Blue reduction rate for the cytotoxicity of phyllodulcin.

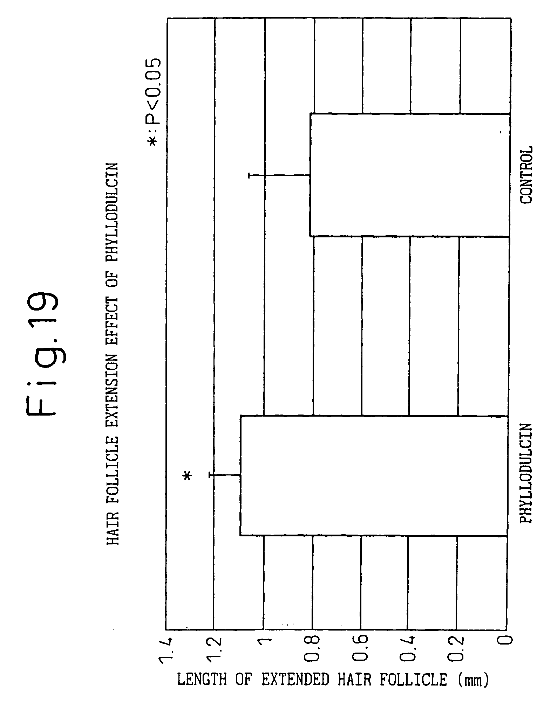

Fig. 19 shows the result of determination of the effect of promoting hair follicle extension carried out on phyllodulcin.
Fig. 20 is a photograph showing the result of investigation on the extension of hair when phyllodulcin was added to the isolated human hair.

### Best Mode for Carrying Out the Invention

The growth cycle of human hair undergoes a growth phase of 5-6 years, a regression phase of 2-3 weeks, and a resting phase of 2-3 months, leading to hair loss, and soon new hair emerges and starts its growth phase. Firstly, the present inventors have experimentally demonstrated that TGF-β2 induces and starts the regression phase, i.e. curtails the growth phase. Thus, by subjecting the hair follicles during the growth phase obtained from the human scalp to an organ-culture in the presence or absence of TGF-β2, it was confirmed, morphological changes occur similar to that in the transition to the regression phase in the natural state when they were cultured in the presence of TGF-β2.

Also, the distribution of TGF-β2 in human hair follicles during the growth phase and human hair follicles during the regression phase were observed in immunohistological staining using anti-TGF-β2 antibody, and thus it was found that the expression and/or distribution of TGF-β2 is more pronounced in the hair follicle during the regression phase as compared to the hair follicle during the growth phase (Working Example 1).

Furthermore, human hair follicles were organ-cultured in the presence or absence of anti-TGF-β2 antibody and fetuin that are known to be antagonists of TGF-β2, and the extension of hair was measured. As a result, in the presence of anti-TGF-β2 antibody or fetuin, the extension of hair was more pronounced as compared to that in their absence, confirming that antagonists of TGF-β2 neutralize TGF-β2 and inhibits transition to the regression phase (Working Example 2).

The present inventors have further confirmed in Working Example 3 that apoptosis is occurring in hair matrix cells when hair follicles involuted by staining human regression phase hair follicles with the TUNEL method.

Then, caspase-3 was observed in each phase of the hair cycle, and caspase-3 was confirmed to be present throughout all phases of the hair cycle, which suggested that caspase-3 is functioning in apoptosis.

Accordingly, in the organ culture of human hair follicles, carbobenzoxy-valyl-alanyl-β-methyl-aspar-1-yl fluoromethane (z-VAD-fmk) that is known to be a caspase-3 inhibitor was added in order to observe the extension of hair and the preservation of morphology of the hair bulb. The result confirmed that z-VAD-fmk that is a caspase-3 inhibitor promotes the extension of hair, and is involved in the retention of morphology of the hair bulb, and thus it was found that caspase-3 inhibitors extend the growth phase of the hair cycle and exhibit the effect of revitalizing hair.

The foregoing revealed that hair loss occurs via a cascade comprising the enhanced production of a male hormone (DHT) by 5αR-II, the enhanced production of TGF-β2 by the male hormone, the activation of caspase by TGF-β2, and the progress of apoptosis by the activation of caspase.

Thus, hair tonics for preventing hair loss preferably contain the combination of two or more of an inhibitor of 5αR-II activity, an inhibitor of effect of TGF-β2, and an inhibitor of caspases, especially the caspase-3 that is present at the furthermost downstream point in the cascade.

Furthermore, hair tonics for preventing hair loss can be screened by the selection of either of an inhibitor of 5αR-II activity, the selection of an inhibitor of effect of TGF-β2, and the selection of an inhibitor of caspases, especially caspase-3 that is present in the furthermost downstream of the cascade, especially the combination of two or more of them.

The inhibition of 5αR-II activity can be determined as follows. 25 µl of the enzyme 5αR-II solution, 25 µl of a sample, and a radioactive testosterone solution (³H-testosterone (1 x 10⁶ dpm), 1 µm testosterone, glucose-6-phosphate (5 mM), glucose-6-phosphate dehydrogenase (2 units/ml), and NADP (1 mM)) are incubated at 37°C for 40 minutes and then, after stopping the reaction, the reaction products are separated by thin layer chromatography, and dpm is counted.

The inhibition of effect of TGF-β2 can be determined as follows. Utilizing the fact that TGP-β2 induces the production of PAI-1, cells (e.g., dermal papilla cells) are cultured in the coexistence of TGF-β2 and the test substance, PAI-1 in the medium is determined, and to determine whether or not PAI-1 was inhibited as compared to the control.

The inhibition of caspase-3 can be determined as follows. As a reaction buffer solution, 25 mM HEPES (pH 7.5), for example, is used, to which 10% sucrose, 0.1% 3-[(3-cholamidopropyl)dimethylammonio]-1-propane sulfate (CHAPS) and 5 mM dithiothreitol (DTT) are included. In addition, as a substrate, a synthesized fluorogenic substrate, acetyl-Asp-Glu-Val-Asp-methylcoumarin amide (Ac-DEVD-MCA), is added to a final concentration of 0.5 mM. Then, a test sample is added to this solution, which is incubated for a fixed time at a fixed temperature (e.g., 30 minutes at 37°C), and the reaction is stopped by a reaction stopping solution, followed by the determination with a fluorometer (excitation: 355 nm; emission: 460 nm).

The apoptosis inhibition of the present invention can be determined as follows. First, cultured mouse epithelial keratinocytes are prepared. Then, culturing of the epithelial keratinocytes of newborn BALB/c mice is continued in a high nutrient medium (DHEM medium) containing 10% fetal calf serum (FCS) and 5-fold concentrated amino acids and vitamins. This results in the dominant growth of large cells having obscure boundaries between the cells, and the formation of high cell-density areas that are layered and covered with keratin on parts of surface of the culture. The above large cells having obscure boundaries between the cells are removed with trypsin treatment, and this procedure is repeated for a few months, and cell populations exhibiting layers are collected. They are Pam212 cells. At the time of passage, 0.05% trypsin and 0.1% EDTA are used to detach and disperse the cell, and the confluent cells are divided in 1:10 and plated onto a new dish, which again reaches confluence in 2-3 days.

Morphologically, cells that have the characteristics of the primary culture of keratinocytes, i.e. circular to polygonal, than the normal epithelium grow in colonies in monolayers. Some tend to overlap. When they become overconfluent, the whole cell layer tends to detach from or fall off, the dish.

Keratin having the same molecular weight (67, 59, 55, 50 kDa) as the newborn mouse epithelium is expressed. The ornithine decarboxylase activity is higher than the primary culture of epithelial keratinocytes, and is markedly enhanced by a phorbol ester treatment. When they are inoculated into syngeneic mice, the mice develop squamous cell carcinoma with a probability of 100%, and acquires the growth ability in agar. The growth and differentiation of the primary culture of epithelial keratinocytes are controlled by the concentration of Ca²⁺ and the change is not pronounced in Pam212 cells. However, Ca²⁺ at low concentrations (<0.1 mM) does not form intercellular bridges, and changes in the cytoskeleton may be seen.

The above cells develop apoptosis and form floating dead cells in the presence of tumor necrosis factor α (TNF-α) and cyclohexamide (CHX). Thus, in the screening of apoptosis inhibitors, cultured mouse epithelial keratinocytes, e.g. Pam212 cells, are cultured in a 10%FCS + DMEM medium containing TNF-α (10 ng/ml) and CHX (10 µg/ml), whereupon a test substance is added or not added, and the occurrence of floating dead cells is compared.

The growth cycle of human hair undergoes the growth phase of 5-6 years, the regression phase of 2-3 weeks, and the resting phase of 2-3 months, leading to hair loss, and soon new hair emerges and starts its growth phase. Firstly, the present inventors have experimentally demonstrated that TGF-β2 induces and starts the regression phase, i.e. curtails the growth phase. Thus, by subjecting the hair follicles during the growth phase obtained from the human scalp to an organ-culture in the presence or absence of TGF-β2, it was confirmed that morphological changes similar to that in the transition to the regression phase in the natural state occur when they were cultured in the presence of TGF-β2.

Also, the distribution of TGF-β2 in human hair follicles during the growth phase and human hair follicles during the regression phase was observed in immunohistological staining using anti-TGF-β2 antibody, and thus it was found that the expression and/or distribution of TGF-β2 is more pronounced in the hair follicle during the regression phase as compared to the hair follicle during the growth phase (Reference Example 1).

Furthermore, human hair follicles were organ-cultured in the presence or absence of anti-TGF-β2 antibody and fetuin that are known to be antagonists of TGF-β2, and the extension of hair was measured. As a result, in the presence of anti-TGF-β2 antibody and fetuin, the extension of hair was more pronounced as compared to that in their absence, confirming that antagonists of TGF-β2 neutralize TGF-β2 and inhibits transition to the regression phase (Reference Example 2).

The present inventors have further confirmed in Reference Example 3 that apoptosis is occurring in hair matrix cells when hair follicles involute by staining human regression phase hair follicles with the TUNEL method.

Then, caspase-3 was observed in each phase of the hair cycle, and caspase-3 was confirmed to be present throughout all phases of the hair cycle, which suggested that caspase-3 is functioning in apoptosis.

Accordingly, in the organ culture of human hair follicles, carbobenzoxy-valyl-alanyl-β-methyl-aspar-1-yl-fluoromethane (z-VAD-fmk) that is known to be a caspase-3 inhibitor was added in order to observe the extension of hair and the retention of morphology of the hair bulb. The result confirmed that z-VAD-fmk that is a caspase-3 inhibitor promotes the extension of hair and is involved in the retention of morphology of the hair bulb, and thus it was found that caspase-3 inhibitors extends the growth phase of the hair cycle and exhibits the effect of revitalizing hair.

The foregoing revealed that hair loss occurs via a cascade comprising the enhanced production of a male hormone (DHT) by 5αR-II, the enhanced production of TGF-β2 by the male hormone, the activation of caspase by TGF-β2, and the progress of apoptosis by the activation of caspase.

Thus, the hair tonics of the present invention can be selected as inhibitors against caspases, especially caspase-3 that is present in the furthermost downstream of the cascade. As such caspase-3 inhibitors, there can be illustrated extracts of Cuachalalate, extracts of Filipendula multijuga, extracts of black tea, extracts of oolong tea, extracts of Coix lacryma-jobi, extracts of Lithospermum erythorhizon siebolt Zuccarini, extracts of Indigofera tinctoria Linn, extracts of Cassia fistula, extracts of Lamium album, extracts of tormentilla, extracts of grape leaves, and extracts of linden.

The inhibition of caspase-3 can be determined as follows. As a reaction buffer solution, 25 mM HEPES (pH 7.5), for example, is used, to which 10% sucrose, 0.1% 3-[(3-cholamidopropyl)dimethylaminonio]-1-propane sulfate (CHAPS) and 5 mM dithiothreitol (DTT) are included. In addition, as a substrate, a synthesized fluorogenic substrate, acetyl-Asp-Glu-Val-Asp-methylcoumarin amide (Ac-DEVD-MCA) is added to a final concentration of 0.5 mM. Then, a test sample is added to this solution, which is incubated for a fixed time at a fixed temperature (e.g., 30 minutes at 37°C), and the reaction is stopped by a reaction stopping solution followed by the determination with a fluorometer (excitation: 355 nm; emission: 460 nm).

The apoptosis inhibition of the present invention can be determined as follows. First, cultured mouse epithelial keratinocytes are prepared. Thus, culturing of the epithelial keratinocytes of newborn BALB/c mice is continued in a high nutrient medium (DHEM medium) containing 10% fetal calf serum (FCS) and a 5-fold concentrated amino acids and vitamins.

This results in the dominant growth of large cells having obscure boundaries between the cells, and the formation of high cell-density areas that are layered and covered with keratin on parts of surface of the culture. The above large cells having obscure boundaries between the cells are removed with trypsin treatment, and this procedure is repeated for a few months, and cell populations exhibiting layers are collected. They are Pam212 cells. At the time of passage, 0.05% trypsin and 0.1% EDTA are used to detach and disperse the cell, and the confluent cells are divided in 1:10 and plated onto a new dish, which again reaches confluence in 2-3 days.

Morphologically, cells that have the characteristics of the primary culture of keratinocytes, i.e. circular to polygonal, than the normal epithelium grow in colonies in monolayers. Some tend to overlap. When then become overconfluent, the whole cell layer tends to detach from, or fall off, the dish.

Keratin having the same molecular weight (67, 59, 55, 50 kDa) as the newborn mouse epithelium is expressed. The ornithine decarboxylase activity is higher than the primary culture of epithelial keratinocytes, and is markedly enhanced by a phorbol ester treatment. When they are inoculated into syngeneic mice, the mice develop squamous cell carcinoma with a probability of 100%, and acquire the growth ability in agar. The growth and differentiation of the primary culture of epithelial keratinocytes are controlled by the concentration of Ca²⁺, the change is not pronounced in Pam212 cells. However, Ca²⁺ at low concentrations does not form intercellular bridges, and changes in the cytoskeleton may be seen.

The above cells develop apoptosis and form floating dead cells in the presence of tumor necrosis factor α (TNF-α) and cyclohexamide (CHX). Thus, in the screening of apoptosis inhibitors, cultured mouse epithelial keratinocytes, e.g. Pam212 cells, are cultured in a DMEM + 10%FCS medium containing TNF-α (10 ng/ml) and CHX (10 µg/ml), whereupon a test substance is added or not added, and the occurrence of floating dead cells is compared.

As hair loss inhibitors that have a caspase-3 inhibitory activity and an apoptosis inhibitory activity, there can be illustrated extracts of Cuachalalate, extracts of Filipendula multijuga, extracts of black tea, extracts of oolong tea, extracts of Coix lacryma-jobi, and extracts of Lithospermum erythorhizon siebolt Zuccarini. Extracts of Cuachalalate are most preferred.

In order to obtain plant extracts, preferably a plant material to be extracted is dried, and, as needed, fragmented or crushed. As extracting agents, there can be used water, organic solvents, and mixed solvents of water-miscible organic solvents and water, with mixtures of water-miscible organic solvents and water being particularly preferred. As organic solvents, there can be mentioned ethanol, methanol and the like. Aqueous solutions of ethanol are preferred, with a 70% aqueous solution of ethanol being particularly preferred. As extraction concentrations, temperature from room temperature to the boiling point of the extracting agent may be used, with 20°C to 37°C being particularly preferred. Extraction is performed for three hours to seven days.

Extraction solutions can be dried by removing solvents according to a standard method of evaporation under reduced pressure, etc. Also, when non-toxic solvents such as water and aqueous solutions of ethanol are used as extraction solvents, the extraction solutions may be used, as the ingredients of hair loss inhibitors, as they are or after appropriate concentration.

The present invention also relates to hair tonic cosmetics, inhibitors of transition into the regression phase, therapeutic agents for liver cirrhosis, or therapeutic agents for nephritis, obtained by the above second screening method, and the hair tonic cosmetics among them comprises as an active ingredient isocoumarins (compounds), specifically phyllodulcin. Other inhibitors of transition into the regression phase, therapeutic agents for liver cirrhosis, or therapeutic agents for nephritis also contain isocoumarins (compounds), especially phyllodulcin as an active ingredient.

Hereinbelow, the best mode for carrying out the present invention will be explained. First, a novel screening method from which the present invention originated is specifically explained.

The growth cycle of human hair undergoes the growth phase of 5-6 years, the regression phase of 2-3 weeks, and the resting phase of 2-3 months, leading to hair loss, and soon new hair emerges and starts its growth phase.

Firstly, the present inventors have experimentally demonstrated that TGF-β2 induces and starts the regression phase, i.e. curtails the growth phase. Thus, by subjecting the hair follicles during the growth phase obtained from the human scalp to an organ-culture in the presence or absence of TGF-β2, it was confirmed that morphological changes occur similar to that in transition to the regression phase in the natural state when they were cultured in the presence of TGF-β2. Also, the distribution of TGF-β2 in human hair follicles during the growth phase and human hair follicles during the regression phase were observed in immunohistological staining using anti-TGF-β2 antibody, and thus it was found that the expression and/or distribution of TGF-β2 is more pronounced in the hair follicle during the regression phase as compared to the hair follicle during the growth phase.

Furthermore, human hair follicles were organ-cultured in the presence or absence of anti-TGF-β2 antibody and fetuin that are known to be antagonists of TGF-β2, and the extension of hair was measured. As a result, in the presence of anti-TGF-β2 antibody and fetuin, the extension of hair was more pronounced as compared to that in their absence, confirming that antagonists of TGF-β2 neutralize TGF-β2 and inhibit transition to the regression phase.

Then, in order to find means to obtain easily an antagonistic effect against TGF-β2, dermal papilla cells were cultured in media containing various concentrations of TGF-β2, and by determining PAI-1 in the culture, it was confirmed that TGF-β2 promotes the production of PAI-1 in dermal papilla cells. Furthermore, it was confirmed that, in the culture of dermal papilla cells in the presence of TGF-β2, the addition of a TGF-β2 antagonist, anti-TGF-β2 antibody, inhibits the enhanced production of PAI-1. From these experiments, it was confirmed that, by determining PAI-1 in the culture of dermal papilla cells according to a standard method, the presence or absence, quantity, activity etc. can be easily determined.

Based on the above novel findings, the present inventors have developed a screening method. Thus, when dermal papilla cells were cultured in the presence of TGF-(32 and various test substances, the amount of PAI-1 in the culture was determined, and the extension by an organ culture of hair follicles was determined, test substances that lower the value of PAI-1 were confirmed to promote the extension of hair, confirming the effectiveness of this screening method.

According to this method, cells (dermal papilla cells, fibroblasts) that have an ability of producing PAI-1 may be cultured in the presence of TGF-β2 and test substances, and the amount of PAI-1 in the culture may be determined. In this case, when the measured value of PAI-1 is significantly lower than that of the control to which no test substance was added, the test substance is judged to be TGF-β2 (antagonist), and is considered to be a candidate hair tonic.

The dermal papilla cells used in this method are isolated from human scalp according to the method of Messenger (Messenger AG: BR J Dermatol 110:685, 1984). Culturing thereof employs Dulbecco's Modified Eagle's Medium (DMEM) supplemented with 10% fetal bovine serum (FBS) etc. As dermal papilla cells, there can be used immortalized cultured dermal papilla cells. Immontalized dermal papilla cells may be obtained at large quantities by culturing optionally, and has an advantage that a large number of test substances can be screened simultaneously or sequentially in a few runs.

Immortalized dermal papilla cells may be established by infecting SV40-T antigen to normal dermal papilla cells, culturing them for a few weeks, then by cloning a multitude of clones from the growing clones, and then continuing the culture of clones that have excellent propagating ability and that are similar to normal dermal papilla cells in morphology and properties. For example, they can be established according to the method proposed by Handa et al. (Patent Application No. 9-271927).

Dermal papilla cells can be cultured in a standard medium for culturing animal cells. The concentration of TGF-β2 in the medium for screening is about 0.01 ng/ml to 100 ng/ml, and culturing is usually performed at 37°C in the presence of CO2. The culturing time till the determination of PAI-1 is about 8 to 72 hours.

Fibroblasts can also be isolated, cultured, and immortalized cells thereof can be established according to a standard method. PAI-1 is determined according to a standard method, e.g. a sandwich ELISA (enzyme-linked immunosorbent assay) using anti-PAI-1 antibody.

The present invention was developed by the present inventors, and the effectiveness of hair tonic cosmetics or inhibitors of transition into the regression phase of the present invention was confirmed by using a screening method of which effectiveness had been confirmed, and the effectiveness of the screening method will further be explained in Working Examples 10-13.

As described above, the present invention provides hair tonic cosmetics, inhibitors of transition into the regression phase, therapeutic agents for liver cirrhosis, or therapeutic agents for nephritis comprising as an active ingredient isocoumarins, hydroisocoumarins among others, specifically phyllodulcin, and hair tonic cosmetics among them may be provided in the form of pharmaceutical drugs, quasi-pharmaceutical drugs, or cosmetics.

The dosage forms are not specifically limited as long as they exhibit the effect of the present invention, and any dosage form may be selected. For example, there may be mentioned liquids, emulsions, ointments, creams, gels, aerosols, mousse and the like. Specifically, there can be mentioned tonics, lotions, conditioners, scalp treatment, shampoos, rinses, and the like.

In these formulations, ingredients normally blended in pharmaceuticals and cosmetics may be blended in addition to isocoumarins including phyllodulcin claimed in the present invention within the scope that the effect of the present invention is not deteriorated. For example, as medical ingredients, there can be mentioned those drugs that have an effect of promoting blood circulation such as extracts of swertia herb, vitamin E and derivatives thereof, nicotinic acid esters such as nicotinic acid benzylester and the like.

As agents that promote blood circulation by the effect of local stimulation, there can be mentioned capsicum tincture, cantharis tincture, camphor, vanillylamide nonylate, and the like. As agents that have the effect of revitalizing hair follicles, there can be mentioned hinokitiol, placenta extracts, photosensitizers, pantothenic acid, and derivatives thereof. As agents that have an anti-male hormone effect, there can be mentioned hormones such as estradiol, ethinyl estradiol, and estrone. As agents that have anti-seborrhea effect, there can be mentioned sulfur, thioxolone, vitamin B₆ and the like.

In addition, there can be mentioned salicylic acid, resorcin and the like having the keratolytic effect and bactericidal effect for preventing the generation of dandruff, glycyrrhizinic acid and derivatives thereof and menthol for preventing inflammation in the scalp, amino acids such as serine, methionine and arginine, vitamins such as biotin, extracts from crude drugs and the like for nutrient supply to hair follicles and revitalization of enzyme activity.

Also, plant extracts such as althaea, coix seed, peppermint, Rumex japonicus, capsicum, aloe, mugwort, sagebrush, oryza, Viticis Fructus, Rosmarinus officinalis, drynaria, Scotch bloom, gentiana, Chinese red sage, sponge gourd, platycodon root, pine, sophora root, Japanese angelica, safflower, Japanese barberry, areca nut, eucalyptus, prunella spike, akebia stem, Achyranthes root, bupleurum root, tea, licorice, hop, Chrysanthemum, senega, sesame, cnidium rhizome, Polygonum multiflorum Thunb., pueraria root, Rosa rugosa var. pleva., saffron, rosemary, rehmannia root, and mallow can be blended.

Also, vasodilators such as alkoxycarbonylpyridine N-oxide, carpronium chloride and acetylcholine derivatives; cutaneous hyperfunctioning agents such as cepharanthin; antibacterial agents such as hexachlorophene, benzalkonium chloride, cetylpyridinium chloride, undecylenic acid, trichlorocarbanilide, bithionol, phenol and isopropylmetylphenol; zinc and derivatives thereof; lactic acid and alkyl esters thereof; organic acids such as citric acid, succinic acid and malic acid; protease inhibitors such as tranexamic acid; and the like can be blended. In addition, drugs such as cyclosporins, oxendolone, diazoxide and minoxidil can also be blended.

Also, alcohols such as ethanol and isopropyl alcohol; polyvalent alcohols such as glycerol, propylene glycol and polyethylene glycol; oily ingredients such as higher fatty acids, higher alcohols, hydrocarbon oil, natural oils and fats, ester oils and silicone oils; surfactants; flavoring agens; chelating agents; humectants such as 1,3-butylene glycol, hyaluronic acid and derivatives thereof, maltitol, atelocollagen and sodium lactate; thickening agents such as quince mucilage, carboxyvinyl polymers and xanthan gum; macromolecular compounds; antioxidants; ultraviolet absorbers: coloring agents; water; stabilizers; and the like, which are usually blended in drugs or cosmetics can also be blended.

Isocoumarins that are an active ingredient of preventive or therapeutic agents of the hair etc. of the present invention can be blended in pharmaceuticals, quasi-pharmaceuticals, cosmetics and the like, and especially are useful as therapeutic agents of skin disorders that improves skin disorders such as acne. Examples of dosage forms of skin medicaments include ointments, lotions, milky lotions, creams, mask, gel, foundations, lip sticks, face colors, skin washes, bath additives and the like, and are not specifically limited as long as the dosage forms are applicable as skin medicaments. In the skin medicaments, ingredients commonly blended in skin medicaments such as kaurenes can be blended within the range that does not cause any problems.

Compounds containing the isocoumarins of the present invention can also be used as pharmaceutical compositions and cosmetic compositions other than those mentioned above. Pharmaceutical compositions may be either oral or parenteral type, and the dosage forms may be any form such as liquids, syrups, tablets, powders, granules, capsules, suppositories, and injections.

In formulating the dosage forms, as needed, commonly used additives such as diluents, excipients, binders, disintegrants, lubricants, colorants, corrigents, stabilizers, solubilizers, and pH adjusting agents may be used in the manufacture according to a standard method. For example, for preparing liquid formulations, physiological saline, 1,3-butylene glycol etc. may be used as a diluent or a carrier.

The concentration of isocoumarin compounds blended in the composition of the present invention, which may be determined as appropriate depending on the shape of the composition or the intended use etc., is usually 0.001-2% by weight. When it is less than 0.001%, the effect of the present invention can be hardly attained, and when it exceeds 2% by weight, it is pharmaceutically undesirable.

As to the dosage, which may also be determined depending on the shape of the composition or the intended use etc., 0.001-100 mg is applied or sprayed onto the scalp or the skin once or a few times a day in the case of hair tonic cosmetics or drugs for external use.

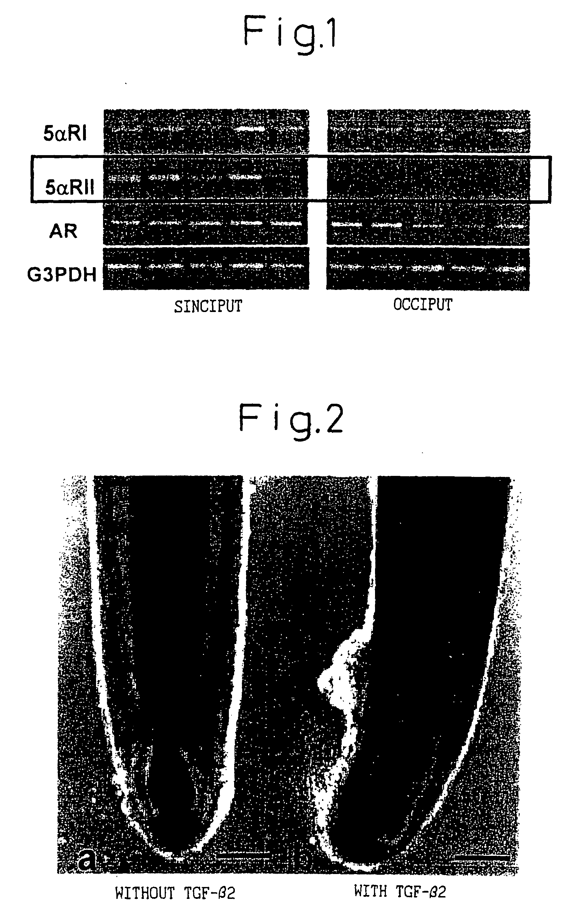

The present invention will now be specifically explained with reference to Examples.
Working Example 1. Confirmation that 5αR-II is only expressed in dermal papilla cells obtained from the sinciput region of a patient with male patterned hair loss

mRNA of cells obtained from the sinciput region or the occipital region of a patient with male patterned hair loss was PCR-amplified with primers for amplifying 5αR-I, 5αR-II, AR, and G3PPH, and the result detected is shown in Fig. 1. The result confirmed that 5αR-II is only expressed in the sinciput region.
Working Example 2. Confirmation that TGF-β2 promotes the start of the regression phase

### (1) Promotion of the start of the regression phase by TGF-β2

Three antibiotics of penicillin, streptomycin and fungizone were added to the Williams E medium (Gibco), and the medium to which 10 ng/ml hydrocortisone, 10 µg/ml insulin, 10 ng/ml sodium pentaselenate and 10 µg/ml transferrin were further added was made the insulin-containing medium, and the medium to which they were not added was made the basal medium, and were used in a human hair follicle organ culture.

Using a microtome under a stereoscopic microscope, hair follicles in the growth phase were isolated from a human scalp. The isolated hair follicles were washed in the basal medium followed by the measurement of length, and then immersed in the insulin-containing medium (a 24-well microplate was used; 1 ml per well), and was cultured overnight at 37°C in an atmospheric condition containing 5% CO₂. After measuring the length again, hair follicles of which length extension was 0.25 mm or longer and of which morphology in the growth phase was retained were selected, and were divided in groups of 10 to 12 pieces so that the length extension may become equal. For each group, the medium was changed to one containing test substances, and the culturing was continued under the above gas phase condition.

The extension of hair follicles was determined over time by inserting a micrometer into the eyepiece of an inverted microscope. Photographs of the whole hair follicles and hair bulbs were taken by a camera connected to the inverted microscope.

On the second day of culturing, the medium was changed to one in which TGF-β2 was added to a final concentration of 50 ng/ml, and the culturing was continued for five days while observing the extension of hair follicles and morphological changes. As shown in Fig. 2, in hair follicles to which TGF-β2 was added on the sixth day of culturing, the regression phase-like morphological changes were promoted as compared to the non-added control. For other growth factors and cytokines, no phenomena were observed in which the regression phase-like morphological changes were promoted. Thus, it was estimated that TGF-β2 has an effect of promoting the regression phase.

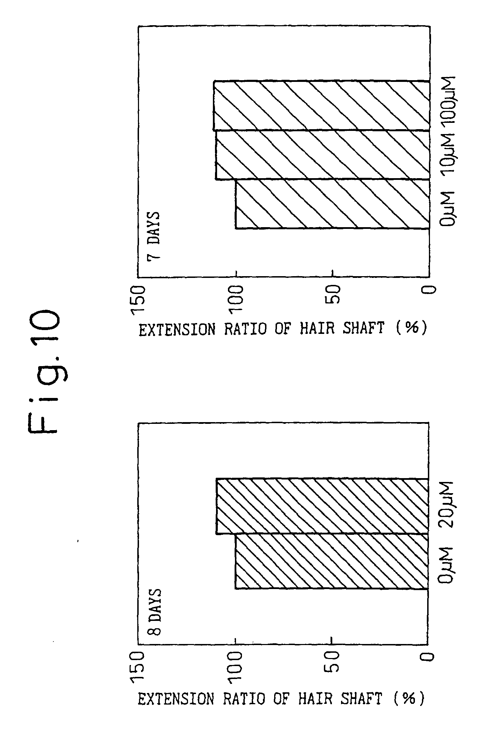

Morphological changes of hair follicles during transition from the growth phase to the regression phase in the normal hair are shown in Fig. 10. From the comparison of Fig. 2 and Fig. 10, it is clear that transition to the regression phase was promoted by TGF-β2.

### (2) Localization of TGF-β2 in human hair follicles

After washing a human scalp tissue or organ-cultured hair follicles in PBS, they were fixed in 4% paraformaldehyde-phosphate buffer (pH 7.2) for 4 hours, dehydrated in an ethanol series, and embedded in paraffin, and then tissue sections of 5 µm in thickness were prepared.

In order to elucidate the role of TGF-β2 in human hair follicles, the localization of TGF-β2 in human hair follicles was investigated. The localization of TGF-β2 in human hair follicles was observed by immunostaining of human scalp tissue sections with anti-TGF-β2 antibody.

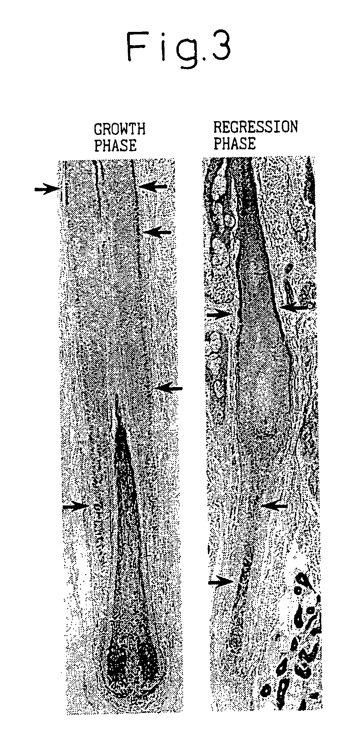

Using human scalp tissue sections that were deparaffin treated and hydrophilicity treated with the ethanol series, and using anti-TGF-β2 antibody (Santa Cruz) as the primary antibody and peroxidase-labelled anti-rabbit IgG as the secondary antibody, the sites of TGF-β2 in human hair follicles were immunohistochemically stained by the avidin-biotin-peroxidase complex method. As shown in Fig. 3, TGF-β2 (immunostaining thereof) was observed in the outermost layer of the outer root sheath in the hair follicles of the late growth phase. On the other hand, in the hair follicle in the late regression phase, TGF-β2 (immunostaining thereof) was observed in the cells in the outermost layer of the outer root sheath and the regressing epithelial cells in the upper part of the dermal papilla. This suggested that TGF-β2 is working in the late regression phase.

### (3) Localization of TGF-β2 in the early regression phase of human hair follicles

As the growth of hair matrix cells decreases and ceases eventually when hair follicles enter the regression phase, it is believed that substances that regulate the growth of hair matrix cells are working in the early regression phase. TGF-β2 is known to strongly inhibit the growth of epithelial cells, and it is likely that TGF-β2 works at the early regression phase to stop the growth of hair matrix cells and to induce the regression phase. In order to prove this possibility, it is necessary to elucidate the localization of TGF-β at the early regression phase.

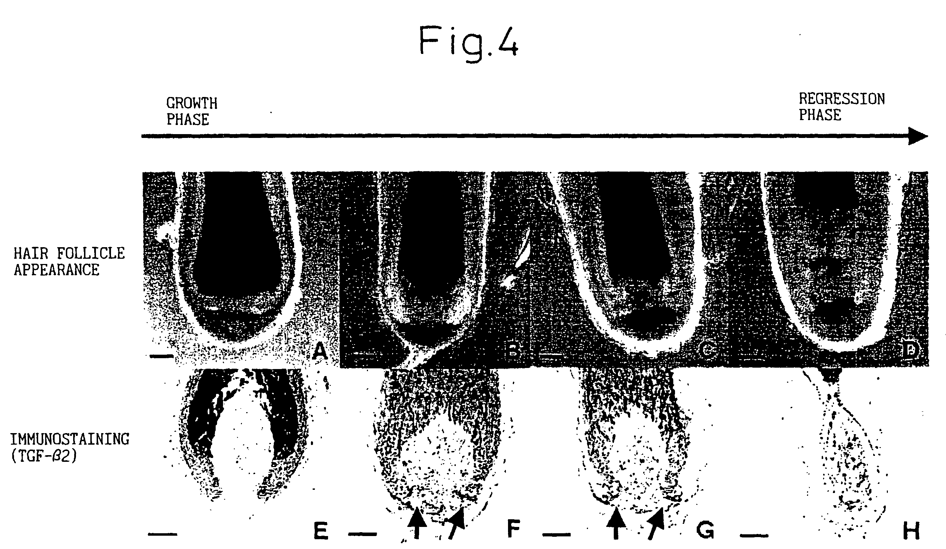

Though it is very difficult to locate hair follicles at the early regression phase in the human scalp tissue section, TGF-β2 was stained to indicate changes at the early regression phase by analyzing over 1000 successive sections obtained from five patients with hair loss. As a result, as shown in Fig. 4, very little TGF-β2 (immunostaining thereof) was observed in hair matrix and dermal papilla in the hair follicles that completely retained the morphology of the growth phase. On the other hand, strong immunostaining of TGF-β2 was observed in the boundary portion of the hair matrix and the dermal papilla in the hair follicles that slightly exhibited the morphology similar to that of the regression phase. This demonstrated the year when TGF-β2 acts to induce the regression phase.

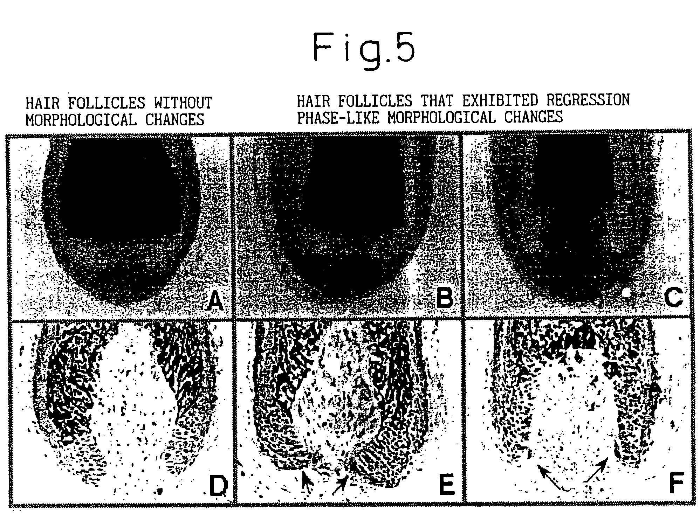

In the organ culture of hair follicles, when hair follicle sections in the growth phase are cultured in the insulin-containing medium, the morphology of the growth phase is retained for about two weeks whereas, when cultured in the basal medium containing no insulin, hair follicles change to morphology similar to that in the regression phase. Thus, by culturing hair follicles in the growth phase in the basal medium containing no insulin, and by examining the localization of TGF-β2 in hair follicles for which slight morphological changes were observed, it can be estimated whether or not TGF-β2 is working in the early regression phase. Thus, after being subjected to organ culture only for one day, hair follicles that retained the morphology in the growth phase and hair follicles that exhibited slight morphological changes similar to those of hair follicles in the regression phase were immunostained with anti-TGF-β2 antibody. As a result, it was observed that changes similar to those in Fig. 4 are actually occurring. This is shown in Fig. 5.
Working Example 3. Effect of inhibiting transition to the hair regression phase by TGF-β2 inhibition

As, in the human hair cycle, TGF-β2 was thought to act to induce the regression phase, as described above, it is estimated that transition to the regression phase may be prevented or delayed by inhibiting the action of TGF-β2 in hair follicles in the growth phase. Thus, the effect of extending the growth phase of hair by TGF-β2 inhibition can be expected. Specifically, the effect of inhibiting transition to the regression phase of hair was demonstrated by whether hair extension can be promoted or morphological changes similar to those in the regression phase are inhibited when a substance that inhibits the action of TGF-β2 is added in the organ culture of human hair follicles.

### (1) Effect of TGF-β-neutralizing antibody

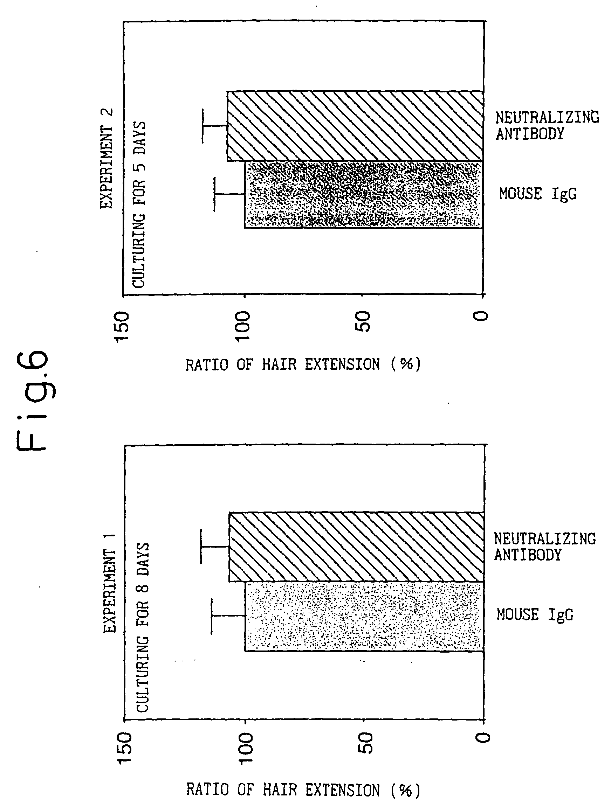

The effect of TGF-β-neutralizing antibody (known to have the activity of neutralizing human TGF-β1 and TGF-β2) of inhibiting transition to the regression phase of hair was demonstrated. The method was performed in accordance with the human hair follicle organ culture method. On the second day of culturing, the medium was changed to one in which anti-TGF-β antibody (manufactured by Genzyme) having the effect of neutralizing TGF-β or the control mouse IgG were added to a final concentration of 20 µg/ml or 100 µg/ml, and culturing was continued for 4-7 days while observing the extension and morphological changes of hair follicles. As shown in Fig. 6, a tendency was seen that the addition of TGF-β-neutralizing antibody promotes the extension of hair. Also, as shown in Table 1, the addition of TGF-β-neutralizing antibody increases the ratio of hair follicles in which the morphology of hair bulb area was retained.

**Table 1:**

| Ratio of hair follicles of which morphology was retained | | |
|---|---|---|
| | Experiment 1 (day 8) | Experiment 2 (day 5) |
| Control (mouse IgG) | 36.4% (4/11) | 55.6% (5/9) |
| Anti-TGF-β antibody 20µg/ml | 54.5% (6/11) | - |
| Anti-TGF-β antibody 100µg/ml | - | 77.8% (7/9) |

### (2) Effect of a sialoglycoprotein fetuin

A sialoglycoprotein fetuin (molecular weight 48,400) is a substance occurring in mammal fetal serum and the blood of adults with various diseases (especially with trauma). As fetuin has an amino acid sequence and a secondary structure similar to those of TGF-β receptor, and acts as an antagonist against TGF-β (Demetriou M. et al., J. Biol. Chem. 271: 12755-12761, 1996), the effect of fetuin of inhibiting transition to the regression phase of hair was demonstrated.

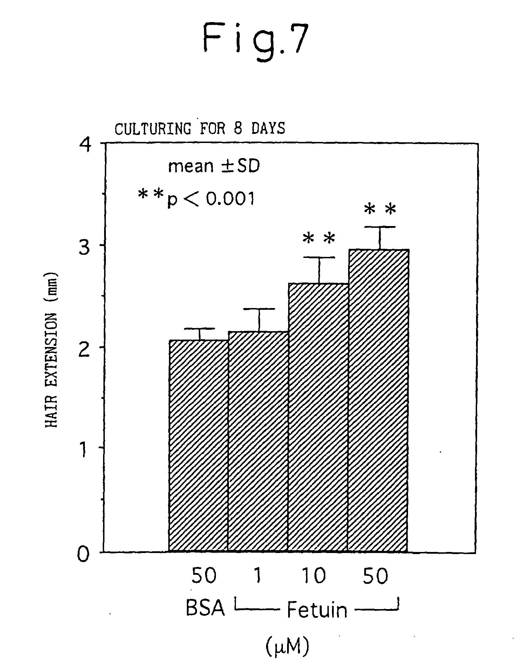

The medium was changed to one in which fetuin (final concentration: 1, 10, 50 µM) or the control bovine serum albumin (final concentration: 50 µM) was added to the basal medium, and culturing was continued for a further 7 days while observing the extension and morphological changes of hair follicles. As shown in Fig. 7, the addition of fetuin significantly extended hair as compared to the addition of the control bovine serum albumin. From these results of TGF-β-neutralizing antibody and fetuin, the effect of inhibiting the transition to the regression phase of hair by the inhibition of TGF-β(2) effect was demonstrated.
Working Example 4. Confirmation of apoptosis in the regression phase hair follicles

After washing a human scalp tissue or organ-cultured hair follicles in PBS, they were fixed in 4% paraformaldehyde-phosphate buffer (pH 7.2) for 4 hours, dehydrated in an ethanol series, and embedded in paraffin, and then tissue sections of 5 µm in thickness were prepared.

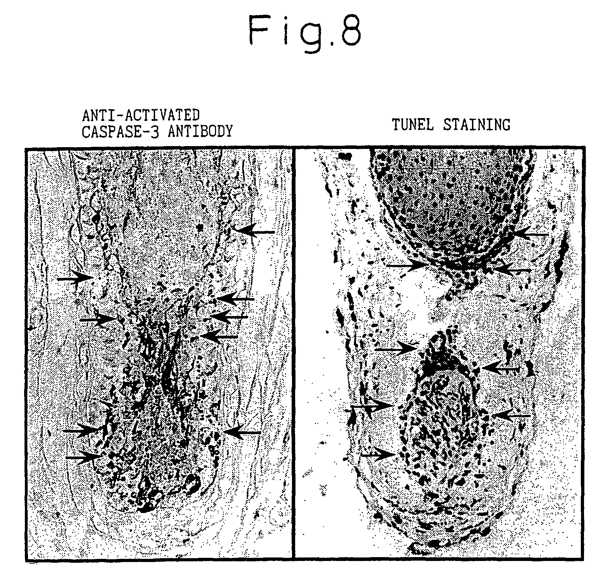

Since hair matrix cells present in the periphery of dermal papilla are stained when human hair follicles in the regression phase are stained by the TUNEL method, it can be seen that apoptosis is occurring in hair matrix cells when hair follicles involute (Fig. 8).
Working Example 5. Distribution of the precursor caspase-3

Caspases are all produced as precursors, and are cleaved by molecules present upstream of the caspase, and thus are activated. Thus, using antibody against precursor caspase-3, the localization of the precursor caspase-3 by hair follicles was investigated. Using human scalp tissue sections that were deparaffin treated and hydrophilicity treated with the ethanol series, and using mouse anti-human precursor caspase-3 antibody (Immunotech) as the primary antibody and peroxidase-labelled mouse IgG as the secondary antibody, the sites of caspase-3 in human hair follicles were immunohistochemically stained by the avidin-biotin-peroxidase complex method.

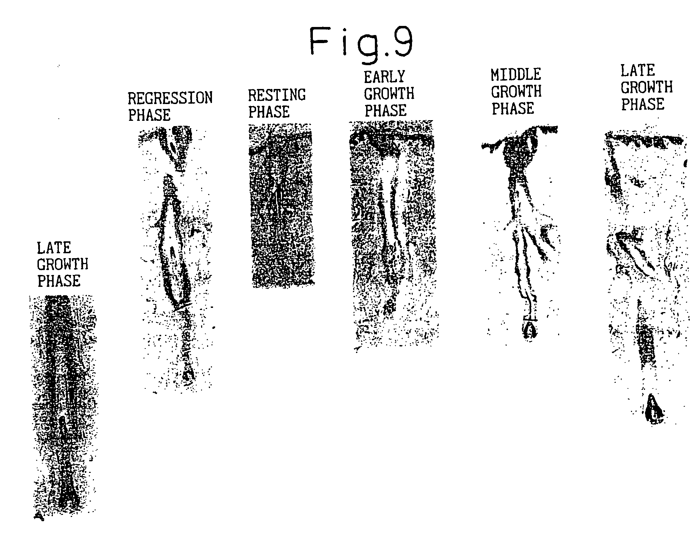

As shown in Fig. 9, the immunostaining of precursor caspase-3 was observed in the entire area of the hair follicular cells throughout the human hair cycle. This suggested that hair follicular cells are always producing caspase-3, and that caspase-3 is also working in the apoptosis of hair follicles.
Working Example 6. Effect of extending the hair growth phase by the inhibition of caspase-3 activity

As hair follicular cells including hair matrix cells are always producing caspase-3, it is believed that when the stimulation of apoptosis is sent to hair follicles and caspase-3 is activated by hair matrix cells in the periphery of dermal papilla, apoptosis is induced in these (hair matrix) cells, with a result that hair follicles lead to regression (hair loss).

From this, it is thought that by inhibiting the activity of caspase-3 and thereby suppressing apoptosis of hair matrix cells and outer root sheath cells, the regression of hair follicles can be prevented or delayed. Thus, the effect of extending the hair growth phase by inhibition of caspase-3 activity can be expected. In order to specifically demonstrate effects, it was investigated whether hair extension is promoted or morphological changes similar to those in the regression phase can be inhibited when a substance that inhibits the activity of caspase-3 was added in the organ culture method of human hair follicles.

Three antibiotics of penicillin, streptomycin and fungizone were added to the Williams E medium (Gibco), and the medium to which 10 ng/ml hydrocortisone, 10 µg/ml insulin, 10 ng/ml sodium pentaselenate and 10 µg/ml transferrin were further added was made the insulin-containing medium, and the medium to which they were not added was made the basal medium, and were used in a human hair follicle organ culture.

Using a microtome under a stereoscopic microscope, hair follicles in the growth phase were isolated from a human scalp. The isolated hair follicles were washed in the basal medium followed by the measurement of length, and then immersed in the insulin-containing medium (a 24-well microplate was used; 1 ml per well), and was cultured overnight at 37°C in an atmospheric condition containing 5% CO₂. After measuring the length again, hair follicles of which length extension was 0.25 mm or longer and of which morphology in the growth phase was retained were selected, and were divided in groups of 10 to 12 pieces so that length extension may become equal. For each group, the medium was changed to one containing test substances, and the culturing was continued under the above gas phase condition.

The extension of hair follicles was determined with time by inserting a micrometer into the eyepiece of an inverted microscope. Photographs of the whole hair follicles and hair bulbs were taken by a camera connected to the inverted microscope.

Carbobenzoxy-valyl-alanyl-β-methyl-aspar-1-yl-fluoromethane (z-VAD-fmk) is known to inhibit almost all caspases. Accordingly, the effect of z-VAD-fmk of extending hair in the growth phase was demonstrated by the organ culture method. The medium was changed to one in which z-VAD-fmk (final concentration: 1, 10, 100 µM) was dissolved in DMSO or the control DMSO was added to the basal medium, and culturing was continued for further 7 days while observing the extension and morphological changes of hair follicles. As shown in Fig. 10, the addition of z-VAD-fmk significantly extended hair as compared to the addition of the control DMSO. The addition of z-VAD-fmk also increased the ratio of hair follicles in which the morphology of the hair bulb portions was retained (Table 2).

**Table 2 :**

| Ratio of hair follicles in which the morphology of the hair bulb portions was retained | | |
|---|---|---|
| | Experiment 2 (day 6) | Experiment 2 (day 4) |
| Control | 20% (2/10) | 12.5% (1/8) |
| z-VAD 10 µM | - (6/10) | 37.5 % (3/8) |
| z-VAD 20 µM | 60% (6/10) | - |
| z-VAD 100 µM | - | 50.5% (4/8) |

Working Example 7. Screening of caspase-3 inhibitors

To 16 µl of a solution in which 0.5 mM of substrate Ac-DEVD-MCA (acetyl-Asp-Glu-Val-Asp-methylcoumarin amide) was added to 25 mM HEPES buffer (pH 7.5) containing 10% sucrose, 0.1% CHAPS, and 5 mM DTT, and 2 µl of test samples and 2 µl of the caspase-3 enzyme solution were added, and a total of 20 µl of reaction mixture was incubated at 37°C for 30 minutes, and after adding 200 µl of the reaction stopping solution, it was measured by a fluorometer (excitation: 355 nm; emission: 460 nm).

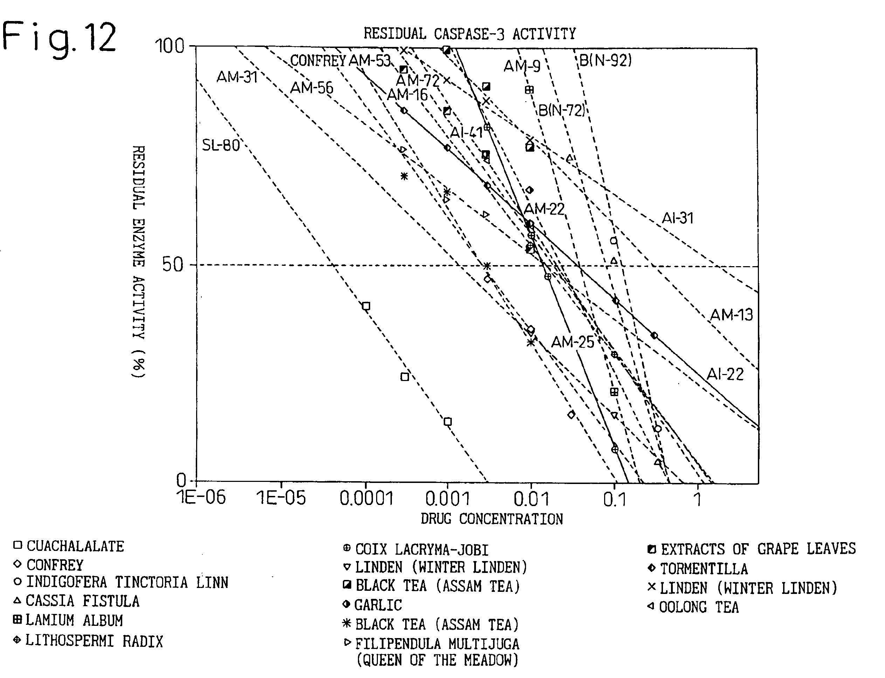

As test samples, there were used extracts of Cuachalalate, confrey, Indigofera tinctoria Linn, Cassia fistula, Lamium album, Lithospermum erythorhizon siebolt Zuccarini, Coix lacryma-jobi, black tea, tormentilla, Filipendula multijuga, grape leaves, linden, and oolong tea. The result is shown in Fig. 12.
Working Example 8. Screening of apoptosis inhibitors

The epithelial keratinocytes of newborn BALB/c mice were cultured in a high nutrient medium (DHEM medium) containing 10% FCS and 5-fold concentrated amino acids and vitamins, with a result that the dominant growth of large cells having obscure boundaries between the cells was seen, but the formation of high cell-density areas that are layered and covered with keratin was seen. The large cells having obscure boundaries between the cells were removed with trypsin treatment, and this procedure was repeated for a few months, and cell populations exhibiting layers were isolated and made Pam212 cells.

The above Pam212 cells were cultured in a DMEM + 10% FCS medium containing 10 ng/ml TNF-α and 10 µg/ml of cyclohexamide (CHX), which was made an apoptosis generation experimental system. Pam212 cells develop apoptosis in a medium containing TNF-α and CHS, but not when cultured in a medium containing no such additives.

As test samples, extracts of sweet hydrangea leaves, extracts of confrey, extracts of Filipendula multijuga, extracts of Cuachalalate, a mixture of extracts of sweet hydrangea leaves and extracts of confrey, a mixture of extracts of sweet hydrangea leaves and extracts of Filipendula multijuga, and a mixture of extracts of sweet hydrangea leaves and extracts of Cuachalalate were used. Furthermore, one in which no TNF α or CHX was added or no test samples were added to the culture system was made "control", and those in which test samples were added to the culture system to which no TNF α or CHX was added were made, respectively, "Filipendula multijuga control", "sweet hydrangea leaf control", and "Cuachalalate control." The result is shown in Table 3.

**Table 3**

| Apoptosis-inhibiting effect by Pam212 cells | |
|---|---|
| Test sample | Ratio of apoptosis cells % |
| Sweet hydrangea leaf extracts + confrey extracts | 100 |
| Sweet hydrangea leaf extracts + Filipendula multijuga extracts | 60 |
| Sweet hydrangea leaf extracts + Cuachalalate extracts | 60 |
| Confrey extracts | 117 |
| Filipendula multijuga extracts | 55 |
| Sweet hydrangea leaf extracts | 75 |
| Cuachalalate extracts | 63 |
| Filipendula multijuga control | 5 |
| Sweet hydrangea leaf control | 5 |
| Cuachalalate control | 7 |
| Control | 6 |
| TNF α and CHX only control | 100 |

As a result, apoptosis-inhibiting effects were observed in extracts of Filipendula multijuga and extracts of Cuachalalate for which a caspase-3-inhibiting effect was observed in Working Example 6, and extracts of sweet hydrangea leaves known to have a TGF β2-inhibiting effect.

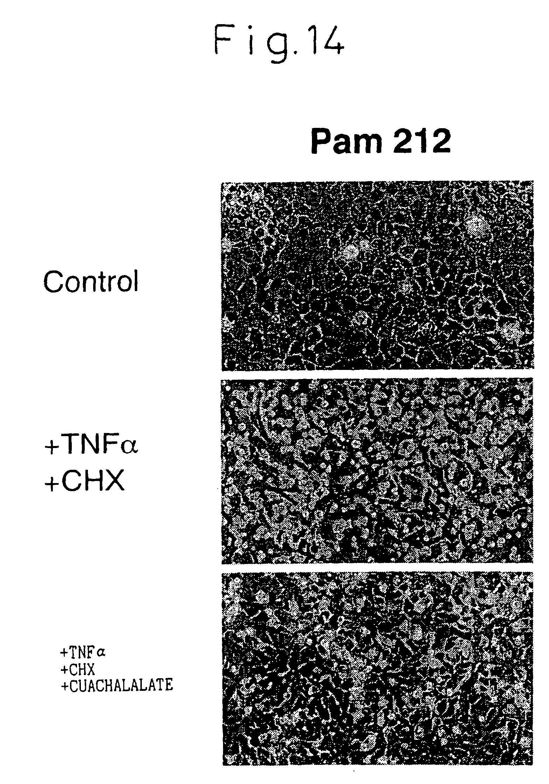

In Fig. 14, for example, the upper part is the control Pam212 cells, the middle part is Pam212 cells cultured with the addition of TNF α and CHX, indicating that apoptosis is occurring. The lower part is when extracts of Cuachalalate were added, and it can be seen therefrom that apoptosis that occurred in the middle part has been inhibited by extracts of Cuachalalate.

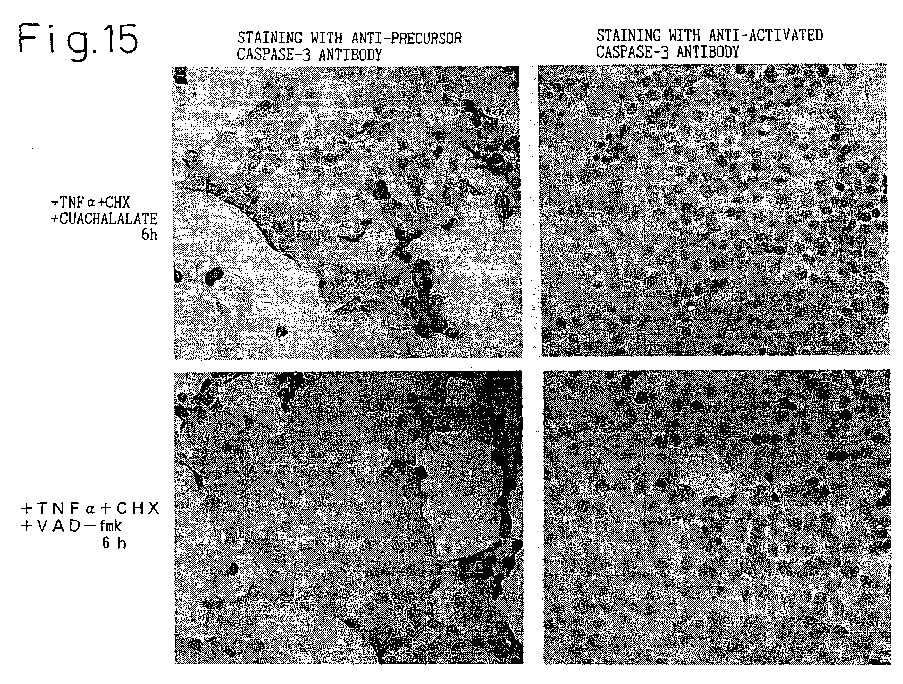

Furthermore, Fig. 15 shows the result in which after extracts of Cuachalalate or VAD-fmk was added under the condition (TNF α and CHX were added) that causes apoptosis and then incubated for six hours, the precursor caspase-3 and the activated caspase-3 were detected using anti-precursor caspase-3 antibody and anti-activated caspase-3 antibody. The precursor caspase-3 was detected in almost all cells, whereas activated caspase-3 was not detected. This indicates that extracts of Cuachalalate and VAD-fmk completely inhibit the activation of the precursor caspase-3.
Working Example 9. Effect of combined use of fetuin having a TGF-β2-inhibiting effect and a caspase-3 inhibitor z-VAD-fmk

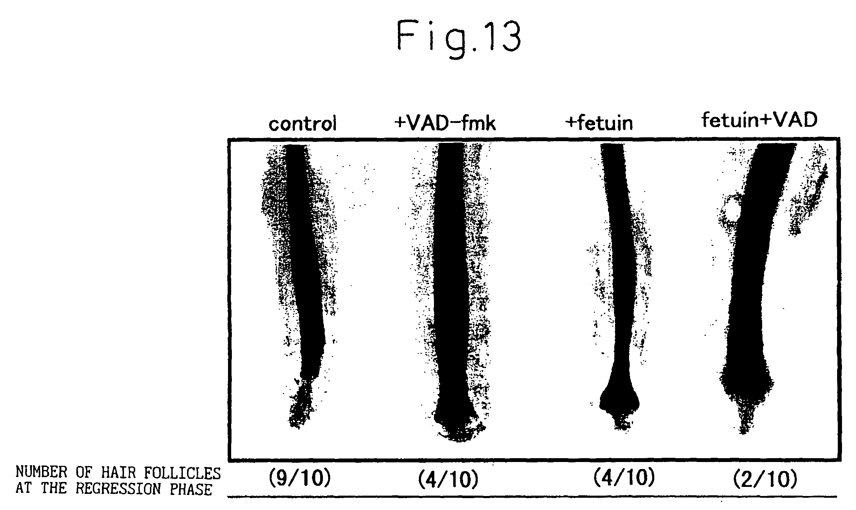

Photographs are shown in Fig. 13 of hair follicles that were cultured for one week in the presence of fetuin having a TGF-β2-inhibiting effect, a caspase-3 inhibitor z-VAD-fmk, or both. It can be seen that the morphology of hair follicles are well retained when the two are present.

As shown under the photographs, the ratio of hair follicles that exhibit regression phase-like changes clearly decreased in the presence of the two.
Working Example 10. Confirmation that TGF-β2 promotes the start of the regression phase The above confirmation was made by the following study.

### (1) Confirmation study that TGF-β2 promotes the start of the regression phase

Three antibiotics of penicillin, streptomycin and fungizone were added to the Williams E medium (Gibco), and the medium to which 10 ng/ml hydrocortisone, 10 µg/ml insulin, 10 ng/ml sodium pentaselenate and 10 µg/ml transferrin were further added was made the insulin-containing medium, and the medium to which they were not added was made the basal medium, and were used in a human hair follicle organ culture.

Under a stereoscopic microscope, hair follicles in the growth phase were isolated from a human scalp. The isolated hair follicles were washed in the basal medium followed by the measurement of length, and then immersed in the insulin-containing medium (a 24-well microplate was used; 1 ml per well), and were cultured overnight at 37°C in an atmospheric condition containing 5% CO₂. After measuring the length again, hair follicles of which length extension was 0.25 mm or longer and of which morphology in the growth phase was retained were selected, and were divided in groups of 10 to 12 pieces so that the length extension may become equal.

For each group, the medium was changed to one containing test substances, and the culturing was continued under the above gas phase condition. The extension of hair follicles was determined with time by inserting a micrometer to the eyepiece of an inverted microscope. Photographs of the whole hair follicles and hair bulbs were taken by a camera connected to the inverted microscope. On the second day of culturing, the medium was changed to one in which TGF-β2 was added to the basal medium to a final concentration of 50 ng/ml, and culturing was continued for further five days while observing the extension and morphological changes of hair follicles.

On the sixth day of culturing, regression phase-like morphological changes were promoted in the hair follicles to which TGF-β2 had been added as compared to the non-added control. In other growth factors and cytokines, the above phenomenon in which regression phase-like morphological changes are promoted was not observed. Thus, it was believed that TGF-β2 had the effect of promoting the regression phase.

Also, morphological changes in hair follicles in transition from the growth phase to the regression phase in the natural hair were observed. From the comparison result of the two, it is clear that transition to the regression phase is promoted by TGF-β2.

### (2) Confirmation study of TGF-β2 localization in human hair follicles

After washing a human scalp tissue or organ-cultured hair follicles in PBS, they were fixed in 4% paraformaldehyde-phosphate buffer (pH 7.2) for 4 hours, dehydrated in an ethanol series, and embedded in paraffin, and then tissue sections of 5 µm in thickness were prepared.

In order to elucidate the role of TGF-β2 in human hair follicles, the localization of TGF-β2 in human hair follicles was investigated. The localization of TGF-β2 in human hair follicles was observed by immunostaining of human scalp tissue sections with anti-TGF-β2 antibody.

Using human scalp tissue sections that were deparaffin treated and hydrophilicity treated with the ethanol series, and using anti-TGF-β2 antibody (Santa Cruz) as the primary antibody and peroxidase-labelled anti-rabbit IgG as the secondary antibody, the sites of TGF-β2 in human hair follicles were immunohistochemically stained by the avidin-biotin-peroxidase complex method. As a result, TGF-β2 (immunostaining thereof) was observed in the outermost layer of the outer root sheath in the hair follicles of the late growth phase. On the other hand, in the hair follicle in the late regression phase, TGF-β2 (immunostaining thereof) was observed in the cells in the outermost layer of the outer root sheath and the regressing epithelial cells in the upper part of the papilla pili. This suggested that TGF-β2 is working in the late regression phase.

### (3) Confirmation study of TGF-β2 localization in the early regression phase of human hair follicles

In the organ culture of hair follicles, when hair follicle sections in the growth phase are cultured in the insulin-containing medium, the morphology of the growth phase is retained for about two weeks, whereas when cultured in the basal medium containing no insulin, hair follicles change to morphology similar to those in the regression phase.

Thus, by culturing hair follicles in the growth phase in the basal medium containing no insulin, and by examining the localization of TGF-β2 in hair follicles for which slight morphological changes were observed, it can be estimated whether or not TGF-β2 is working in the regression phase. Thus, after being subjected to organ culture only for one day, hair follicles that retained the morphology of the growth phase and hair follicles that exhibited slight morphological changes similar to those of hair follicles in the regression phase were immunostained with anti-TGF-β2.

As a result, in the hair follicles that retained completely the growth phase morphology, little TGF-β2 (immunostaining thereof) was observed in hair matrix cells or dermal papilla.

On the other hand, in hair follicles that exhibited slight morphological changes similar to those of hair follicles in the regression phase, strong immunostaining of TGF-β2 was noted in the boundary portion of the hair matrix and the dermal papilla. This revealed that TGF-β2 is working in vitro as well, and induces the regression phase.
Working Example 11. Confirmation of effect of inhibiting transition to the regression phase of hair by TGF-β2 inhibition

The above result indicated that TGF-β2 works in the human hair cycle and induces the regression phase. Thus, it is estimated that transition to the regression phase may be prevented or delayed by inhibiting the action of TGF-β2 in hair follicles in the growth phase. Thus, the effect of extending the growth phase of hair by TGF-β2 inhibition can be expected. Specifically, the effect of inhibiting transition to the regression phase of hair was demonstrated by whether hair extension can be promoted or morphological changes similar to those in the regression phase are inhibited when a substance that inhibits the action of TGF-β2 is added in the organ culture of human hair follicles.

### (1) Confirmation study of effect of TGF-β-neutralizing antibody

The effect of TGF-β-neutralizing antibody (known to have the effect of neutralizing human TGF-β1 and TGF-β2) of inhibiting transition to the regression phase of hair was demonstrated. The method was performed according to the human hair follicle organ culture method. On the second day of culturing, the medium was changed to one in which anti-TGF-β2 antibody (manufactured by Genzyme) having the effect of neutralizing TGF β or the control mouse IgG were added to a final concentration of 20 µg/ml or 100 µg/ml, and culturing was continued for 4-7 days while observing the extension and morphological changes of hair follicles.

From this, a tendency was seen that the addition of TGF-β-neutralizing antibody promotes the extension of hair. Also, as shown in Table 4, the addition of TGF-β-neutralizing antibody increased the ratio of hair follicles in which the morphology of hair bulb area was retained.

**Table 4:**

| The ratio of hair follicles that retained morphology | | |
|---|---|---|
| | Experiment 1 (day 8) | Experiment 2 (day 5) |
| Control (mouse IgG) | 36.4% (4/11) | 55.6% (5/9) |
| Anti-TGF-β antibody 20 µg%/ml | 54.5% (6/11) | - |
| Anti-TGF-β antibody 100µg/ml | - | 77.8% (7/9) |

### (2) Confirmation study of effect of a sialoglycoprotein fetuin

A sialoglycoprotein fetuin (molecular weight 48,400) is a substance occurring in mammal fetal serum and the blood of adults with various diseases (especially with trauma). As fetuin has an amino acid sequence and a secondary structure similar to those of TGF-β receptor, and acts as an antagonist against TGF-β (Demetriou M. et al., J. Biol. Chem. 271: 12755-12761, 1996), the effect of fetuin of inhibiting transition to the regression phase of hair was demonstrated.

The medium was changed to one in which fetuin (final concentration: 1, 10, 50 µM) or the control bovine serum albumin (final concentration: 50 µM) was added to the basal medium, and culturing was continued for further 7 days while observing the extension and morphological changes of hair follicles. The addition of fetuin significantly extended hair as compared to the addition of the control bovine serum albumin. From these results of TGF-β-neutralizing antibody and fetuin, the effect of inhibiting transition to the regression phase of hair by inhibiting the effect of TGF-β2 was demonstrated. Working Example 12. Confirmation that TGF-β-inhibiting effect can be determined by determination of PAI-1

The medium in which 10% bovine fetal serum was added to the DMEM medium to which three antibiotics of penicillin, streptomycin and fungizone had been added was used, and human dermal papilla cells were cultured in a 96-well plate (about 7,500 cells per well). First, dose dependency on TGF-β2 of the increased expression of PAI-1 was examined. At a suitable cell density, the medium was changed to ones containing various concentrations of TGF-β2, and was further cultured for 24 hours. Ten µl of the medium was taken out, and quantitated using a commercially available PAI-1 determination kit (TintElize PAI-1: biopool). The result indicated an increase in PAI-1 in a dose dependent manner of TGF-β2.

When 20 µg/ml on TGF-β-neutralizing antibody had been added simultaneously with TGF-β2, the increase in PAI-1 by TGF-β2 was completely inhibited. This result demonstrates that in a coexistence of TGF-β and a substance that offsets the effect thereof, an increase in PAI-1 is inhibited, indicating that the present method of evaluation is suitable for screening substances that inhibit the effect of TGF-β.
Working Example 13. Confirmation study on the ingredients contained in sweet hydrangea leaves

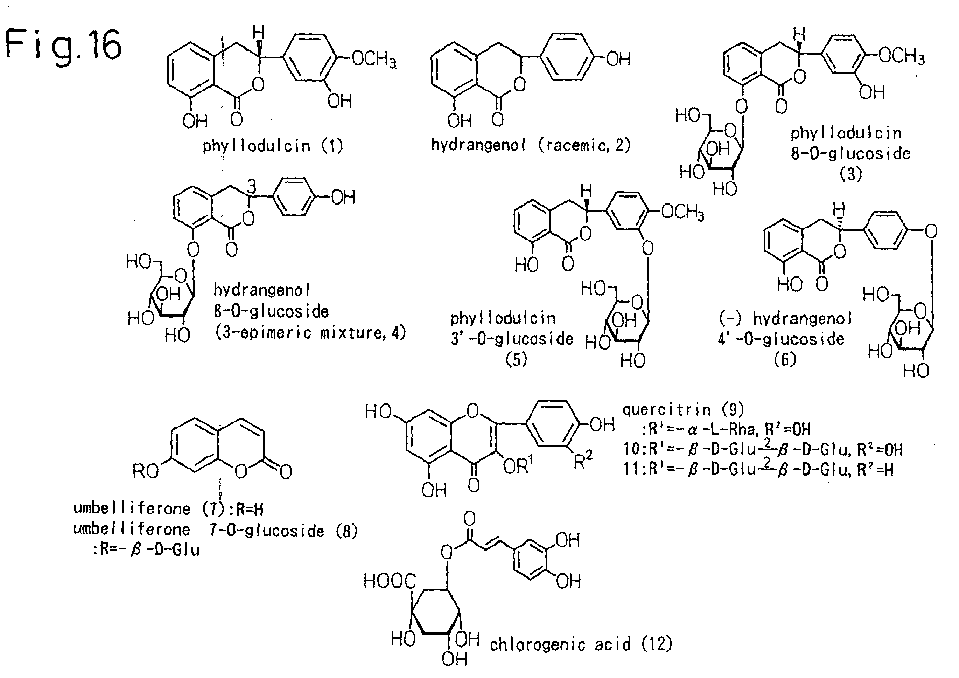

The present inventors have prepared a 50% ethanol solution of extracts of sweet hydrangea leaves, and analyzed the ingredients contained therein, and thereby have confirmed the presence of a variety of substances such as phyllodulcin, hydrangenol, chlorogenic acid, and umbelliferone. Though the present inventors have performed the confirmatory work as described above, it is a known fact that those substances are present in extracts of sweet hydrangea leaves, as shown in Fig. 16.

Then, the present inventors investigated, as described below, whether or not TGF-β2 has the effect of inhibiting the enhanced expression of PAI-1.
Phyllodulcin was dissolved in DMSO at concentrations of 0.05-50 µg/ml and used. After culturing for 24 hours in a medium containing 1 ng/ml TGF-β2, 10 µl of the medium was used to determine the amount of PAI-1. At the end of culturing, the survival of cells was also determined by the Alamar Blue method.

This was performed as described below. Thus, after washing twice in a fresh medium, the medium was changed to one containing 1/10 amount of Alamar Blue (Alamar Bioscience), and was incubated at 37°C (5% CO₂ ) for four hours. After incubation, absorbance at 595 nm and 570 nm was measured by a microplate reader in order to calculate the Alamar Blue reduction rate for each well, which was divided by the Alamar Blue reduction rate for the negative control in which no phyllodulcin samples were added, and the survival of the cells were calculated.

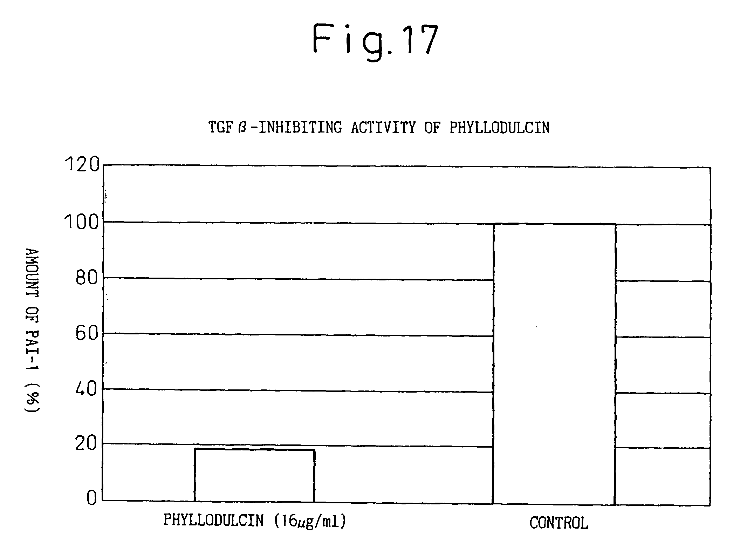

The former result is shown in Fig. 17, which indicates that phyllodulcin inhibits the increase in the expression of PAI-1. Thus, the ordinate of the figure represents the amount of PAI-1, which is 18 when phyllodulcin was added, and is markedly lower than 100 in the case of no addition, indicating an excellent effect of inhibiting the increase in PAI-1.

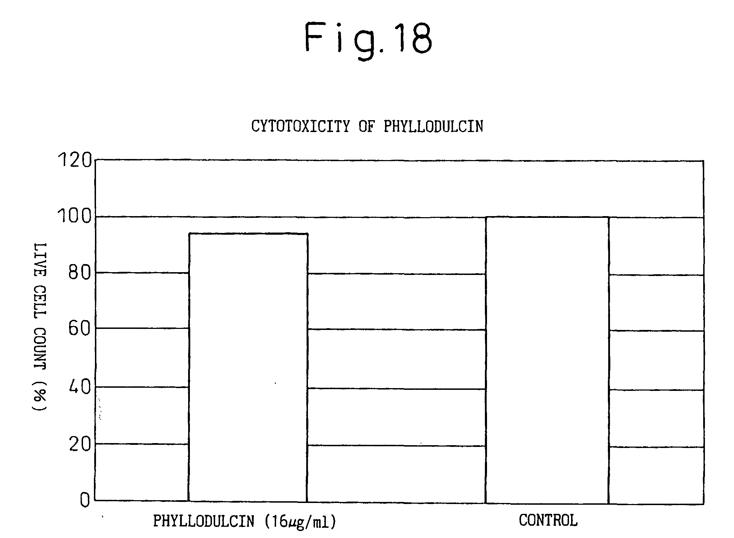

Fig. 18 also illustrates the cytotoxicity of the latter phyllodulcin, which indicates that very little cytotoxicity was observed for phyllodulcin. Thus, the ordinate of the figure represents the Alamar Blue reduction rate (%), and the lower this value, the lower the cytotoxicity is, and the reduction rate when phyllodulcin was added was 95%, which is close to 100% in the case of no addition, indicating a low cytotoxicity.

### (2) Confirmation study of effect of extending hair follicles

The effect of promoting hair extension by phyllodulcin in the organ culture method was demonstrated as described below. Phyllodulcin (Wako Pure Chemicals: standard for assaying crude drugs) was dissolved in DMSO to a concentration of 50 mg/ml. The method used was performed according to the human hair follicle organ culture method. On the second day of culture, the medium was changed to one in which a phyllodulcin solution was added to a final concentration of 0.6 µg/ml, and the culturing was continued for seven days while observing the extension and morphological changes of hair.

As a result, as shown in Fig. 9, hair extension was significantly promoted in the addition group of 0.6 µg/ml phyllodulcin on the eighth day of culturing as compared to the solvent control. This reveals that phyllodulcin selected as a substance for inhibiting the effect of TGF-β2 has the effect of inhibiting transition to the regression phase of hair, indicating the effectiveness of the present invention.

### (3) Confirmation study of effect of extending hair

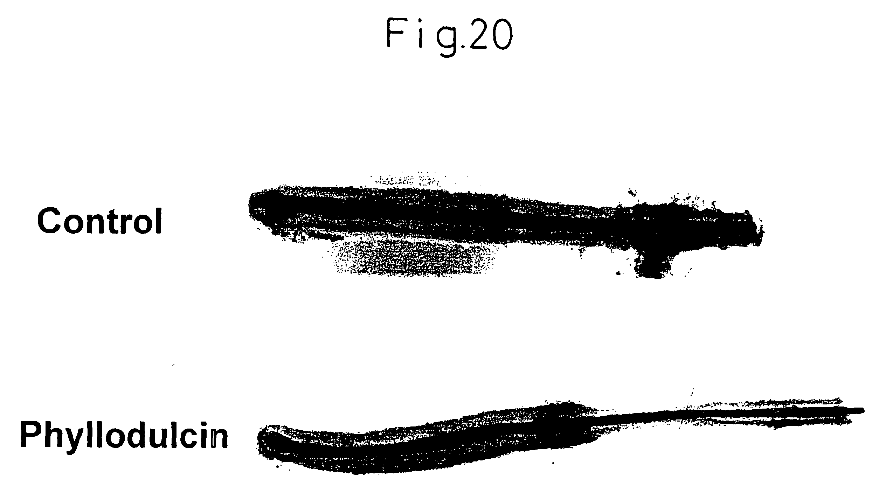

The extension of hair when phyllodulcin was added to the isolated human hair was investigated. The result is as shown in a photograph in Fig. 20. The photograph clearly confirms that when phyllodulcin was added, the hair is extended as compared to the control.

Since it is known that TGF-β2 is one of the causes of liver cirrhosis and nephritis, and phyllodulcin has the effect of inhibiting (antagozing) TGF-β2, it clearly has the property as a therapeutic agent for liver cirrhosis or nephritis. Also, isocoumarin compounds other than phyllodulcin have the effect of inhibiting (antagozing) TGF-β2 as phyllodulcin, and thus are useful as hair tonic cosmetics, an inhibitor of transition to the regression phase of hair, a therapeutic agent for liver cirrhosis or a therapeutic agent for nephritis. Industrial Applicability

By using caspase-3 inhibition and apoptosis inhibition as an index, the screening of hair loss inhibitors can be performed efficiently and with a high precision.

Furthermore, using caspase-3 inhibition and, as desired, apoptosis inhibition as an index, a novel caspase-3 inhibitor can be provided.

The present invention was developed based on the discovery that isocoumarins including phyllodulcin have the characteristics of active ingredients for hair tonic cosmetics, inhibitors of transition to the regression phase of hair, therapeutic agents for liver cirrhosis and therapeutic agents for nephritis, by a novel screening method developed based on the discovery made by the present inventors that transforming growth factor (TGF-β2) promotes transition from the growth phase to the regression phase of the hair growth cycle, and inhibits transition to the regression phase by antagonistic substances (antagonists) of TGF-β2 so as to obtain the effect of hair revitalization.

The present invention permits the effective expression of efficacy as hair tonic cosmetics, an inhibitor of transition to the regression phase of hair, a therapeutic agent for liver cirrhosis and a therapeutic agent for nephritis, by including isocoumarins including phyllodulcin in these compositions. Also, isocoumarins have very little cytotoxicity and thus can exhibit proper performance as hair tonic cosmetics or an inhibitor of transition to the regression phase of hair. Furthermore, this compound is an ingredient of sweet hydrangea leaves that has been used as a beverage, and in this regard also, it is a safely usable active ingredient.

## Claims

1. A hair tonic comprising a combination of at least two of the following:
(a) an inhibitor against 5α reductase type 2 (5αR-II);
(b) an inhibitor against transforming growth factor β2 (TGF-β2); and
(c) an inhibitor against caspase-3.

2. The hair tonic according to claim 1 wherein an inhibitor against transforming growth factor β2 (TGF-β2) and an inhibitor against caspase-3 are combined.

3. The hair tonic according to claim 2 wherein an inhibitor against TGF-β2 is an extract of sweet hydrangea leaves, and an inhibitor against caspase-3 is an extract of Filipendula multijuga or an extract of Cuachalalate.

4. The hair tonic according to claim 2 wherein an inhibitor against TGF-β2 is fetuin, and an inhibitor against caspase-3 is z-VAD-fmk.

5. A method of screening an inhibitor of male patterned hair loss comprising a combination of at least two selections of the following:
(a) selection of an inhibitor against 5α reductase type 2 (5αR-II);
(b) selection of an inhibitor against transforming growth factor β2 (TGF-β2); and
(c) selection of an inhibitor against caspase-3.

6. A caspatase-3 inhibitor comprising as an active ingredient an extract of Indigofera tinctoria Linn, an extract of Cassia fistula, an extract of Lamium album, an extract of tormentilla, an extract of grape leaves, an extract of linden, an extract of Cuachalalate, an extract of Filipendula multijuga, an extract of black tea, an extract of oolong tea, an extract of Coix lacryma-jobi, or an extract of Lithospermum erythorhizon siebolt et Zuccarini.

7. A hair tonic comprising the caspatase inhibitor according to claim 6.

8. A hair tonic according to claim 7 which is a hair loss inhibitor.

9. A hair tonic cosmetics comprising an isocoumarin as an active ingredient.

10. The hair tonic cosmetics according to claim 9 wherein said isocoumarin is phyllodulcin.

11. An inhibitor of transition to the regression phase of hair comprising an isocoumarin as an active ingredient.

12. The inhibitor of transition to the regression phase of hair according to claim 11 wherein said isocoumarin is phyllodulcin.

13. A therapeutic agent for liver cirrhosis comprising an isocoumarin as an active ingredient.

14. The therapeutic agent for liver cirrhosis according to claim 13 wherein said isocoumarin is phyllodulcin.

15. A therapeutic agent for nephritis comprising an isocoumarin as an active ingredient.

16. The therapeutic agent for nephritis according to claim 15 wherein said isocoumarin is phyllodulcin.
